# EUROPEAN PATENT SPECIFICATION

(11) **EP 1 997 482 B1**
(45) Date of publication and mention of the grant of the patent: **06.07.2011**
(21) Application number: 07109085.6
(22) Date of filing: 29.05.2007
(51) Int. Cl.: A61K 9/20, A61K 31/00

(54) **A pharmaceutical composition comprising eszopiclone**
Pharmazeutische Zusammensetzung mit Eszopiclon
Composition pharmaceutique contenant eszopiclone

(43) Date of publication of application: 03.12.2008
(73) Proprietor: LEK Pharmaceuticals d.d., 1526 Ljubljana (SI)
(72) Inventor: Jerala-Strukelj, Zdenka, 4211 Mavcice (SI); Legen, Igor, 1290 Grosuplje (SI); Reven, Sebastjan, 4270 Jesenice (SI)

(56) References cited:
- WO-A-2005/063248
- WO-A-2005/065308
- WO-A-2007/005961
- US-A1- 2007 098 788

## Description

The present invention relates to a stable pharmaceutical composition of eszopiclone.

Eszopiclone or S-zopiclone is a cyclopyrrolone drug with sedative and hypnotic properties. The chemical name of eszopiclone is (+)-5(S)-6-(chloropyridin-2-yik)-7-oxo-6,7-dihydro-5H-pyrrolo[3,4-b]pyrazin-5-yl 4- methylpiperazine-1-carboxylate.

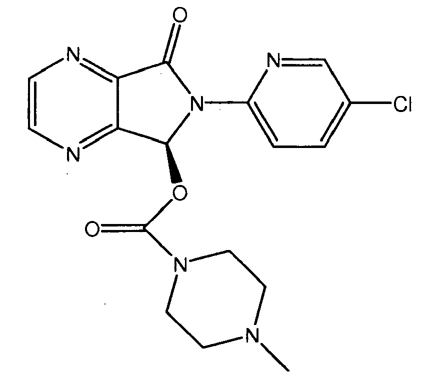

Eszopiclone was first disclosed in EP 268956. A commercially available eszopiclone containing product is Lunesta®.

WO2005063249 provides particles containing the active agent having a diameter from 0.1 mm to 2.5 mm, preferably from 0.5-2 mm. The tablet typically contains a binder, a lubricant, an inert diluent, a preservative, a disintegrant, a dispersing agent.

The eszopiclone particle size used in the pharmaceutical formulations of US2007098788 provides a D90 of 216.1 micrometers, wherein D90 is defined as the distribution where 90 volume percent of the particle are smaller than that size given. In WO200705961 capsules, tablets comprising eszopiclone further include O-DMW succinate salt monohydrate, MCC, basic calcium phosphate anhydrous, croscarmellose sodium, colloidal silicon dioxide, magnesium stearate. Eszopiclone is screened through 80 mesh sieve, thus particles being smaller than 300 micrometers.

Stability is one of the most important factors which determines whether a compound or a mixture of compounds can be developed into a therapeutically useful pharmaceutical product. Impurities that are formed by degradation of the active ingredient during manufacturing of dosage forms or resulting from storage are common impurities in the medicines. The presence of these unwanted chemicals even in small amounts may influence the efficacy and safety of the pharmaceutical products.

Therefore, it is essential, to maintain proper chemical stability of the active substance in dosage form during stability testing and at normal storage conditions.

It was surprisingly found that use of eszopiclone substance with particle size exceeding 100 µm results in significant increase in chemical stability, after the substance is compressed in tablets. With the use of substance of special particle size, we are able to assure that the levels of impurities occuring during manufacturing process and storage, will remain in permitted limits.

During the development of the stable pharmaceutical composition of eszopiclone it was found that eszopiclone substance is stable. However, the stability of eszopiclone is decreased afer the substance is compressed in tablets. Furthermore, it was found that eszopiclone substance is prone to chemical degradation when it is in contact with alkaline species and water. Consequently, to achieve satisfactory chemical stability of eszopiclone in the tablet the excipients that are incorporated in the tablet containing eszopiclone should be carefully selected in order not to exceed pH about 7. Additionally, the water content in the tablet should be minimized - often meaning introducing energy consuming drying in the production of the tablets or employment of very expensive dry excipients.

Surprisingly, we have found that using eszopiclone with particles larger than 100 µm, the satisfactory chemical stability of eszopiclone in the tablets can be achieved even if pH of the excipients is above 7 and even in the presence of higher amount of water in the tablet.

As used herein, the term "eszopiclone particles" means eszopiclone crystals.

As used herein, the term "particles larger than 100 µm", when used in reference to the size of eszopiclone particles, indicates that d (0.9) is higher than 100 µm.

As used herein, the term "particles larger than 200 µm", when used in reference to the size of eszopiclone particles, indicates that d(0.9) is higher than 200 µm.

As used herein, the term "particles larger than 300 µm", when used in reference to the size of eszopiclone particles, indicates that d(0.9) is higher than 300 µm.

As used herein, the term "particles smaller than 500 µm", when used in reference to the size of eszopiclone particles, indicates that d(0.9) is lower than 500 µm.

As used herein, the term "particles smaller than 800 µm", when used in reference to the size of eszopiclone particles, indicates that d(0.9) is lower than 800 µm.

Particle size distribution of eszopiclone was characterized by laser diffraction (Malvern Mastersizer S). Sample cell was small volume sample dispersion cell MS1, presentation was 3NHE, solvent was hexane, stirrer speed was 2000 rpm. The following procedure was used: Fill the sample cell with hexane, add 100 mg of sodium bis (2-ethylhexyl) sulfosuccinate, align the sizer and measure the background. Add sample into the sample cell until proper obscuration is achieved (10-30%). Analyze when the signal from detectors is stable.

Substance containing larger particles has in general lower surface area which may result in decreased water solubility and consequently in decreased biological activity due to impaired absorption from the gastrointestinal tract. However, in case of eszopiclone we observed that the solubility of substance having particles exceeding 200 µm is comparable to solubility of the substance having fine particles. The solubility was measured at pH 6.8, which represents the pH in the small intestine, a primary site of drug absorption. Therefore, no effect on the gastrointestinal absorption can be expected due to larger particles of eszopiclone.

| Active substance | Solubility at pH 6.8 (mg/ml) |
|---|---|
| Eszopiclone with d(0,9) > 200 µm | 0.57 |
| Eszopiclone with d(0,9) = 32.4 µm | 0.44 |

The solubility of eszopiclone in phosphate buffer media at pH 6.8 was studied at room temperature. An excess of sample was placed in the laboratory beaker filled with 50 ml media and mixed on the magnetic stirrer for 60 minutes. Afterwards, the part of the dispersion was taken from the beaker, filtered through the 0.45 µm filter and the concentration of eszopiclone was determined by UV spectroscopy.

Moreover, it was found that there is no significant difference in dissolution profile of tablets comprising large particles of eszopiclone in comparison to tablets comprising small particles of eszopiclone. Consequently, a particle size should not have an effect on a bioavailability of eszopiclone.

Percent of dissolved eszopiclone from Eszopiclon 1 mg tablets in 0.1 M HCl at 50 rpm.

| | Percent of dissolved eszopiclone | |
|---|---|---|
| t (min) | Comparative Example 1 | Example 2 |
| 5 | 94.3 | 97.6 |
| 10 | 103.3 | 98.7 |
| 15 | 103.4 | 98.8 |
| 30 | 103.3 | 98.8 |

Dissolution profiles of tablets described in comparative example 1 (Eszopiclone 1: d(0.5)=14.2 µm and d(0.9)=32.4 µm) and example 2 (Eszopiclone 2: d(0.5)=154.0 µm and d(0.9)=340.0 µm) are similar.

Therefore, the first object of the present invention is related to a stable pharmaceutical composition comprising eszopiclone with particles larger than 100 µm, preferably eszopiclone with particles larger than 200 µm and smaller than 800 µm, more preferably eszopiclone with particles larger than 200 µm and smaller than 500 µm, most preferably eszopiclone with particles larger than 300 µm and smaller than 500 µm.

Pharmaceutical composition of the present invention may contain one or more additional pharmaceutical excipients such as diluents, binders, disintegrants, filers, antiadherents, lubricants, etc.

Suitable diluent, may be selected from the group consisting of lactose, calcium phosphate, microcrystalline cellulose, calcium sulphate. Preferred diluent may be selected from:
a) lactose having a particle size of about 20 to 400 microns and a density of about 0.3 to 0.9 g/ml
b) calcium phosphate having density of about 0.5 to 1.5 g/ml
c) microcrystalline cellulose having a particle size of about 20 to 300 microns and a density of about 0.2 to 0.7 g/ml
d) calcium sulfate having a particle size of about 1 to 200 microns and a density of about 0.6 to 1.3 g/ml.

Suitable binder may be selected from the group consisting of microcrystalline cellulose, hypromellose, povidone. Preferred binder is povidone.

Suitable disintegrant may be selected from the group consisting of croscarmellose sodium, sodium starch glycolate or microcrystalline cellulose. Preferred disintegtrants are croscarmellose sodium and microcrystalline cellulose.

Suitable glidant helps to prevent the adhesion of the tablets to the punches and the dies. It may be selected from the group consisting of starch, colloidal silicon dioxide or talc. Preferred antiadherent is colloidal silicon dioxide.

Suitable lubricant may be selected from the group consisting of stearic acid, talc and magnesium stearate. The lubricant is quite important since it reduces the friction between the die wall and the tablet granule during the compression. Preferred lubricant is magnesium stearate.

In another embodiment the present invention relates to a pharmaceutical composition comprising:
a) 0.5-5% w/w of eszopiclone particles larger than 100 µm,
b) 50-95% w/w of diluent,
c) 0-5% w/w of binder,
d) 0-15% w/w of disintegrant,
e) 0-5% w/w of glidant, and
f) 0-5% w/w of lubricant.

A pharmaceutical compositions according to the present invention is preferably in solid form, including tablets, capsules, caplets, lozenges and sachets. Tablets may be suitably coated (film coated tablets, pills). Capsule formulations may cover both soft and hard capsules. A pharmaceutical compositions according to the present invention is preferably in the form of tablet.

In another embodiment the present invention relates to a process for the preparation of eszopiclone tablets comprising the step of:
a) granulation of eszopiclone with particles larger than 100 µm with a main diluent, a binder, and optionally with a disintegrant,
b) addition of other excipients,
c) compressing a mixture obtained in step b) into tablet cores,
d) optionally, coating the tablet cores.

Eszopiclone with particles larger than 100 µm, is preferably eszopiclone with particles larger than 200 µm and smaller than 800 µm, more preferably eszopiclone with particles larger than 200 µm and smaller than 500 µm, most preferably eszopiclone with particles larger than 300 µm and smaller than 500 µm.

A mixture of indapamide with a main diluent, a binder and optionally a disintegrant is prepared before granulation is performed. Due to very low percent of eszopiclone in the tablets, first a triturate of eszopiclone and a main binder is prepared by mixing eszopiclone and the proper amount of a main binder. After that the another part of the main diluent, a binder and optionally an disintegrant were subsequently added.

In step a) any known method of granulation may be used, preferably a wet granulation. Preferred granulation liquid is demineralised water. Preferably granulation is carried out in a high shear mixer, and drying in a fluid bed dryer.

After step a) obtained dry mixture is optionally sieved.

Preferably in step c) tablet cores are compressed on rotary compressing machine.

In step d) tablet cores may be film coated with appropriate film coating material to protect eszopiclone against light. Any known method for film coating, known in the field of the pharmaceutical technology, may be used,

A pharmaceutical composition of the present invention comprises preferably 1, 2 or 3 mg of eszopiclone.

In another embodiment the present invention relates to use of the pharmaceutical composition of the present invention for the preparation of a medicament with sedative and hypnotic properties.

The following examples illustrate the invention, but do not limit it in any way:

### Comparative Example 1

| ***Core*** | | ***function*** | ***mg*** | ***w*/*w* %** |
|---|---|---|---|---|
| Eszopiclone 1: | d(0.5)=14.2 µm | active | 1.000 | 0.83 |
| | d(0.9)=32.4 µm | | | |
| Lactose monohydrate | | diluent | 88.150 | 73.46 |
| Povidone | | binder | 1.800 | 1.50 |
| Corn starch | | disintegrant | 6.000 | 5.00 |
| Croscarmellose sodium | | disintegrant | 2.400 | 2.00 |
| Demineralised water | | granulation liquid | 23.000 | / |
| Microcrystalline cellulose | | diluent/ binder | 20.000 | 16.67 |
| Colloidal silicon dioxide | | glidant | 0.350 | 0.29 |
| Magnesium stearate | | lubricant | 0.300 | 0.25 |
| Core weight | | | 120.000 | 100.00 |

A batch of 10.000 tablets was prepared with granulation in high shear mixer.
A triturate of eszopiclone and lactose monohydrate was prepared by mixing 10.0 g of eszopiclone and the proper amount of lactose monohydrate. Before the another part of lactose monohydrate was added the dry mixture was sieved through 0.5 mm sieve. To the dry mixture of eszopiclone with lactose monohydrate was subsequently added: 60.0 g of corn starch, 24.0 of croscarmellose sodium and 18.0 g of povidone. The mixture was then blended in a high shear mixer. This dry mix was wet granulated in a high shear with 230.0 g of demineralised water. The wet granules were dried in a fluid bed drier at 50ºC, to a defined moisture content. The dried granules were size reduced in a oscillator (0.8 mm screen) and then mixed with 200.0 g of microcrystalline cellulose and 3.5 g of colloidal silicon dioxide, followed by mixing with 3.0 g of magnesium stearate. The lubricated granules were compressed using round shaped tooling with 7 mm diameter and core weight of 120.0 mg.

### Example 2

| ***Core*** | | ***function*** | ***mg*** | **w/w *%*** |
|---|---|---|---|---|
| Eszopiclone2: | d(0,5)=154,0 µm | active | 1.000 | 0.83 |
| | d(0,9)=340,0 µm | | | |
| Lactose monohydrate | | diluent | 88.150 | 73.46 |
| Povidone | | binder | 1.800 | 1.50 |
| Corn starch | | disintegrant | 6.000 | 5.00 |
| Croscarmellose sodium | | disintegrant | 2.400 | 2.00 |
| Demineralised water | | Granulation liquid | 23.000 | / |
| Microcrystalline cellulose | | diluent/binder | 20.000 | 16.67 |
| Colloidal silicon dioxide | | glidant | 0.350 | 0.29 |
| Magnesium stearate | | lubricant | 0.300 | 0.25 |
| Core weight | | | 120.000 | 100.00 |

The manufacturing procedure is the same as described in example 1, nevertheless eszopiclone with larger particle size was used.

### Example 3

| ***Core*** | | ***function*** | ***mg*** | **w/w %** |
|---|---|---|---|---|
| Eszopiclone3: | d(0,5)=153,0 µm | active | 1.000 | 0.83 |
| | d(0,9)=338,0 µm | | | |
| Lactose monohydrate | | diluent | 76.00 | 63.33 |
| Hypromellose | | binder | 3.200 | 2.67 |
| Croscarmellose sodium | | disintegrant | 1.200 | 1.00 |
| Demineralised water | | granulation liquid | 8.000 | / |
| Microcrystalline cellulose | | diluent/binder | 12.000 | 10.00 |
| Calcium phosphate | | diluent | 26.000 | 21.67 |
| Magnesium stearate | | lubricant | 0.600 | 0.50 |
| Core weight | | | 120.000 | 100.00 |

A batch of 15.000 tablets was prepared with granulation in high shear mixer.
A triturate of eszopiclone and lactose monohydrate was prepared by mixing 15.0 g of eszopiclone and the proper amount of lactose monohydrate. The dry mixture was sieved through 0.5 mm sieve and than another part of lactose monohydrate was added. To the dry mixture of eszopiclone with lactose monohydrate was subsequently added: 18.0 of croscarmellose sodium and 48.0 g of hypromellose. The dry mixture was then blended in a high shear mixer. This dry mix was wet granulated in a high shear with 120.0 g of demineralised water. The wet granules were dried in a fluid bed drier at 50ºC, to a defined moisture content. The dried granules were size reduced in a oscillator (0.8 mm screen) and then mixed with 180.0 g of microcrystalline cellulose and 390.0 g of calcium phosphate, followed by mixing with 9.0 g of magnesium stearate. The lubricated granules were compressed using round shaped tooling with 7 mm diameter and core weight of 120.0 mg.

### Example 4

### Stability testing

Tablets comprising eszopiclone with different particle sizes were prepared and exposed to stress stability testing at 60°C for 14 days, stored in vials. The amount of formed degradation products was compared to the sample of commercially available tablets comprising eszopiclone (Lunesta ®). Results are presented in the Table 1.
The HPLC analysis were performed with a WATERS Acquity UPLC system equipped with a UPLC BEH Shield, 1.7 µm, 50 X 2.1 mm column which was maintained in a column oven at 45 °C. The mobile phase consisted of a mixture of amonium acetate, TEA and acetonitrile with pH = 5.8. The flow rate was 1.0 mL/min, and the detection wavelength was 303 nm.

All impurities eluted within 21 minutes represent the total amount of eszopiclone impurities.

**Table 1.**

| Example no | Particle size distribution (µm) | Time of testing, Testing condition | Total sum of impurities | Max.unknown |
|---|---|---|---|---|
| Substance in a crystal form | | 14 days, 60 °C | < 0,05 % | < 0,05 % |
| Comparative Example 1 | d(0.5)=14.2 µm | 14 days, 60 °C | 2.45 | 0.80 |
| | d(0.9)=32.4 µm | | | |
| Example 2 | d(0.5)=154.0 µm | 14 days, 60 °C | 0.99 | 0.29 |
| | d(0.9)=340.0 µm | | | |
| Example 3 | d(0.5)=153.0 µm | 14 days, 60 °C | 0.51 | 0.13 |
| | d(0.9)=338.0 µm | | | |
| Lunesta 1 mg | | 14 days, 60 °C | 0.81 | 0.23 |

From the results above can be seen that eszopiclone substance is stable when exposed to the elevated temperature of 60 °C for 14 days (substance in a crystal form). The stability of eszopiclone dramatically decreases when compressed into the tablets (Comparative Example 1).

The composition of tablets in example 1 and 2 are completely identical, the only difference is in the particle size distribution of eszopiclone, as we can see in Table 1. The difference between the compositions of example 2 and 3 is in the use of different diluent (calcium phosphate) in the example 3. The particle size of eszopiclone substance in example 2 and 3 is the same.

In comparison to comparative example 1, the decrease in amount of individual impurities and total sum was observed when eszopiclone tablets of examples 2 and 3 were exposed to elevated temperature of 60 °C for 14 days in vials. Even though two most important parameters that affect the stability of eszopiclone, pH value and water content, were not optimal (significantly higher in comparison to the commercially available tablets comprising eszopiclone), comparable or better chemical stability of eszopiclone tablets of examples 2 and 3 in comparison to the Lunesta® is achieved.

## Claims

1. A pharmaceutical composition comprising eszopiclone with particles larger than 300 µm and smaller than 500 µm.

2. A pharmaceutical composition according to claim 1, wherein a pharmaceutical composition is in the form of tablet.

3. A pharmaceutical composition according to claim 1 or claim 2 comprising:
a) 0.5-5% w/w of eszopiclone particles larger than 300 µm and smaller than 500 µm,
b) 50-95% w/w of diluent,
c) 0-5% w/w of binder,
d) 0-15% w/w of disintegrant,
e) 0-5% w/w of glidant, and
f) 0-5% w/w of lubricant.

4. A process for the preparation of eszopiclone tablets comprising the step of:
a) granulation of eszopiclone with particles larger than 300 µm and smaller than 500 µm with a main diluent and a binder,
b) addition of other excipients,
c) compressing a mixture obtained in step b) into tablet cores.

## Patentansprüche

1. Eine pharmazeutische Zusammensetzung, welche Eszopiclone mit Partikeln größer als 300 µm und kleiner als 500 µm enthält.

2. Eine pharmazeutische Zusammensetzung gemäß Anforderung 1, worin die pharmazeutische Zusammensetzung in Form einer Tablette ist.

3. Eine pharmazeutische Zusammensetzung, gemäß Anforderung 1 oder 2, die Folgendes enthält:
a) 0,5-5% w/w Eszopiclone-Partikel größer als 300 µm und kleiner als 500 µm,
b) 50-95% w/w Verdünnungsmittel,
c) 0-5% w/w Bindemittel,
d) 0-15% w/w Sprengmittel,
e) 0-5% w/w Flussregulierungsmittel und
f) 0-5% w/w Gleitmittel.

4. Der Herstellungsvorgang der Eszopiclone-Tabletten umfasst folgende Vorgangsschritte:
a) Eszopiclone - Granulierung mit Partikeln größer als 300 µm und kleiner als 500 µm, zusammen mit dem Hauptverdünnungsmittel und Bindemittel,
b) Hinzufügung anderer Hilfsmittel,
c) einpressen der aus dem Vorgangsschritt b) ergebenen Mischung in die Tablettenkerne.

## Revendications

1. Une composition pharmaceutique comprenant de l'eszopicione avec des particules supérieures à 300 µm et inférieures à 500 µm.

2. Une composition pharmaceutique selon la revendication 1, dans laquelle la composition pharmaceutique est sous forme de comprimé.

3. Une composition pharmaceutique selon la revendication 1 ou la revendication 2 comprenant :
a) 0,5-5 % p/p de particules d'eszopiclone supérieures à 300 µm et inférieures à 500 µm,
b) 50-95 % p/p de diluant,
c) 0-5 % p/p d'agent liant,
d) 0-15 % p/p d'agent délitant,
e) 0-5 % p/p d'agent glissant, et
f) 0-5 % p/p de lubrifiant.

4. Un procédé de préparation de comprimés d'eszopiclone comprenant les étapes de :
a) granulation d'eszopiclone avec des particules supérieures à 300 µm et inférieures à 500 µm avec un diluant principal et un agent liant,
b) addition d'autres excipients,
c) compression du mélange obtenu à l'étape b) en noyaux de comprimés.
